# EUROPEAN PATENT SPECIFICATION

(11) **EP 2 651 367 B1**
(45) Date of publication and mention of the grant of the patent: **16.09.2020**
(21) Application number: 11854112.7
(22) Date of filing: 15.11.2011
(51) Int. Cl.: G16H 40/20, A61J 1/10, A61J 1/18, G06Q 10/08, G06Q 50/22

(54) **MEDICAL PACKAGE, SYSTEM AND METHOD FOR MANAGING MEDICAL PACKAGE**
MEDIZINISCHE VERPACKUNG SOWIE SYSTEM UND VERFAHREN ZUR VERWALTUNG MEDIZINISCHER VERPACKUNGEN
CONDITIONNEMENT MÉDICAL, SYSTÈME ET PROCÉDÉ POUR LA GESTION DE CONDITIONNEMENT MÉDICAL

(30) Priority: 16.12.2010 SE 1001195
(43) Date of publication of application: 23.10.2013
(73) Proprietor: Tridentify AB, 475 40 Hönö (SE)
(72) Inventor: Sandvik, Leif, S-475 40 Hönö (SE); Strandberg, Christian, S-134 38 Gustavsberg (SE); Oskarsson, Thomas, S-421 67 Västra Frölunda (SE); Gramming, Magnus, S-564 32 Bankeryd (SE)
(74) Representative: Valea AB
(86) International application number: PCT/SE2011/051366
(87) International publication number: WO 2012/091655

(56) References cited:
- WO-A2-2006/108026
- WO-A2-2007/014147
- US-A1- 2002 013 523
- US-A1- 2003 069 796
- US-A1- 2004 044 326
- US-A1- 2004 044 326
- US-A1- 2004 166 583
- US-A1- 2004 166 583
- US-A1- 2004 247 016
- US-A1- 2007 001 862
- US-A1- 2007 028 642

## Description

### TECHNICAL AREA

The present invention relates to a medical package, a system and a method for managing the medical package, especially bags containing blood products such as blood and blood plasma, vaccines and packages. The system according to the invention can also be used for managing other types of packaging with a product content that requires special storage conditions for sustainability to be assured.

### BACKGROUND OF INVENTION

During manufacturing, transportation, storage, administration and handling of various medical products, such as blood, blood plasma and vaccines, it is absolutely important that the contents of each package can be identified in a safe manner.

It is also essential to ensure that such medical products are transported and stored under conditions which ensure that they are not adversely affected or destroyed before being administered to a patient or other recipient. Such storage conditions are accomplished usually by the medical package wherever possible be kept in special refrigerators or cold rooms under strict control of temperature, light, moisture, contamination risk, etc.

If one considers that there is a risk that a medical package has been exposed to inappropriate storage conditions, such as an excessively low or high storage temperature, or may have been exposed to contamination, e.g. by the bed of a patient, this package with its product content must be discarded. In the case of blood products has, for example, the EU and national health authorities have started to formulate stronger demands for an improved control over the lifecycle before being administered to the intended recipients.

There are different systems for marking, identification and management of medical products such as blood and blood plasma. This kind of products are generally packaged in bags fitted with a suitable labeling related to product content and other relevant information concerning the product in question. At the site in which the medical products are manufactured and/or filled in their packaging, an operator labels each package with all of data necessary for its use, such as an identification code, date of filling and a specification of the packaged product content. After transportation of the filled package to a unit in which the package must be stored and/or packaged product content must be used, another operator must interpret the labeling and product data listed on the package and enter the data in a local storage system. In the previously known systems, particularly at smaller storages or care and treatment units paper-based and even handwriting-based input of data related to inventory management still exists. An unclear or incomplete manual entry of product data in a storage management system makes it difficult to find required specific package of the product content. An incorrect manual entry of data into a storage management system can at worst results in a risk of confusion between two specific medical packages with various product content and/or different useful life-times.

In the current systems, every movement of packages between various devices, such as between a manufacturing unit and a depot, from a depot and a care facility, or between a cold store and a refrigerator adjacent to an operating theatre, often requires a manual input of several data records for each individual package unit in a variety of storage systems, without requiring users of the systems therefore receive any comprehensive or complete picture of their stock for a specific medical product or the exact storage location of a specific package with the specific product content as required in a specific case. The people who use the current systems are often not accustomed to complex storage management systems and may have difficulty coping with the manual entries of data in the manner required for the systems to function as intended. Besides the fact that incorrect or incomplete entries of data represent a security risk, they can also results in unnecessary discarding of the package. The unclear situation of stocks which easily can follow the current system fragmented structure and manual data entry leads to spending a lot of time for storage inventory and search for specific products and will also result in many unnecessary movements between the various units where medical packages are stored and handled.

In connection with the transport and storage of medical packages, there is, as mentioned earlier, also a risk that the products are subjected to an adverse environmental impact, such as too high temperature, which can results in a shorter shelf lifetime and destruction of a packaged product content. Today's system has often no complete control over which specific medical package that may have been exposed to such adverse environmental impact, which of course is very risky from the product safety point of view. Besides the increased risk for recipients to indicate what the contents of packages should be used for, the lack of control over the remaining life of the individual packages in the system leads to a large number of unnecessary rejections of products. For example, it is common for staff to collect 10 blood bags before surgery and 8 of these blood bags are administered to the patient during surgery. After the operation, 2 of the blood bags are still unused and could perhaps be used for another suitable patient, but with today's system thus must still be discarded for safety reasons because of the lack of knowledge about how the blood in these blood bags have been stored and transported after the bags left the manufacturing or the refilling unit.

U.S. 2004/166583 concerns a method which consists in determining ageing an ageing index of a blood bag, to determine whether the blood bag is or not suitable for transfusion to a patient. The ageing index is regularly calculated at the blood transfusion center from the sample, until it is removed from storage. The present invention differs from this document in two independent elements: the package is provided with a code and other information, and that the indicator is located on the tracking device and not the location of the system where the package is stored, as in this document.

U.S. 2004/044326 relates to a method for detection of bags containing biological fluids. The method comprises the steps of: obtaining a sterile bag for storage of biological fluids; placing a variety of biological fluids in the bag, providing a first information processing device with a data input device and a data transmission device that can write data that has been placed in the information processing device, a chip for storing data in a way that can be read by a second information processing device; provide bag with a readable/writable data storage chip; collecting fluid data on the biological fluid, and entering data, such as fluid data, to the first information processing device for data transfer to the chip. Data storage chip is capable of interaction with the first and second information processing devices. The first information processing unit writes data to data storage chip and the second information processing device can read data from the chip. The procedure of this document does not provide a visual readout of a unique identifier of a package if it would be necessary or no linkage of the unique identity code on the package with a unique device identity of a tracking device that comes with the package. Moreover, it appears that the process according to this document does not allow any visual readout of any marking on the tracking/data chip supplied with the package. Furthermore, it is not clear from the document, that the described procedure would allow the tracking/data storage chip to be cleaned and reused, which is possible with tracking devices in the system according to the present invention.

US2007001862 relates to electronic time-temperature indicators with an RFID output, and other devices and methods by which the thermal history of a complex material, which may not obey a simple exponential Arrhenius law degradation equation, may be monitored, and the subsequent fitness for use of the tracked material may be quickly ascertained. In particular, the invention discloses a rapidly reprogrammable electronic time-temperature RFID tag that may be easily customized with the thermal time-temperature stability profile of an arbitrary material, using electronic data transfer methods. This document is silence of specific housing material of the tracing unit.

### SUMMARY OF THE INVENTION

A first object of the invention is therefore to provide a system for handling medical packages that makes it possible to eliminate the above mentioned problems.

The invention also provides a reliable sensor-based real-time temperature monitoring system for medical products, such as blood bags. The system is versatile and can be used both for the current quality information and monitoring changes over time and show position. The system can ensure quality, traceability and availability of the product/blood all the time. The system is also recyclable, which leads to lower costs and meets the growing demand for waste minimization.

This first objective is achieved with a system according to characterizing portion of claims 1.

The system for handling medical package according to the invention ensures, using the tracking device according to the invention, that packages and data related to their product content are not confused during management of the system. Thanks to the logging of environmental parameters by using the tracking device that comes with each package, a complete verification that all the packages in the system still have an estimated remaining useful lifetime is achieved. The system according to the invention ensures that a required package with a specific product content always can be identified quickly and without confusion, that this specific product content is still useful when it is administered, and reduces the number of unnecessary rejections, as the system operators have a clear indication of the status of product content in a specific package when the package is handled in the system.

A second object of the invention is to provide a system for handling medical package that makes it possible to record the relevant data for a specific new medical package and a new specific product content in the system in a user-friendly and safe manner, and then be able to follow this particular package and this specific product content for the continued management of the system.

This second objective is achieved with a system according to patent claims 2-11.

Thanks to means for reading the unique identity code and other relevant information on a specific package of the access point, means for sensing the unique device identity of the included tracking device and a means to transfer of all this information into a database where all the related information should be associated, the system operators' workload and the risk of incorrect data entry in the system is reduced, since the number of stations where data on package and/or product content must be entered manually into the system by an operator is minimized.

A third object of the invention is to provide a system for handling medical package that allows system users to be able to find a specific package or a specific product content as requested, to follow this specific package's or this specific product content's movement and current location in the system and to obtain information about an estimated expiration date for the specific product content of a specific package, from a selectable spot by one of the users. This third objective is achieved with a system according to patent claims 2-11.

Thanks to the provision of the portal for the system users, the system users can from a selectable spot access all the data required for identification, tracking, positioning, ordering, transportation, quality assessment and decision on the administration of specifically requested product content in a specific package. This reduces the workload for system users and enables a transport vehicle can be sent directly to the current position where the required package or packages, which reduces the amount of unnecessary transport.

The invention also concerns a tracking device for use in a system for handling medical packages in accordance with claim 12.

The invention also relates to a method for managing medical packages in accordance with claim 13.

Other objectives, advantages and features of the present invention will become apparent from the following description, patent claims and the attached drawings

### BRIEF DESCRIPTION OF THE DRAWINGS

In the following some embodiments of the invention are described in more detail, merely as an example and with reference to the attached schematic drawings, where:
Figure 1 is a schematic illustration of a medical package in the form of a blood bag, along with a tracking device as part of a preferred embodiment of the system according to the invention, whereby the displayed tracking device is designed to accompany the blood bag during a period that extends from the input of the blood bag in the system to a possible decision on administration of blood in the blood bag to an intended recipient or a decision on disposal of blood bag,
Figure 2 is a schematic illustration of an access point as part of a preferred embodiment of the system according to the invention, along with a blood bag with an included tracking device according to the invention;
Figure 3 is a schematic block diagram of the structure of a preferred embodiment of the system according to the invention;
Figure 4 illustrates schematically a second embodiment of a tracking device, according to the present invention, and
Figure 5 illustrates schematically example of the electronics in a tracking device, according to the present invention.

### DETAILED DESCRIPTION

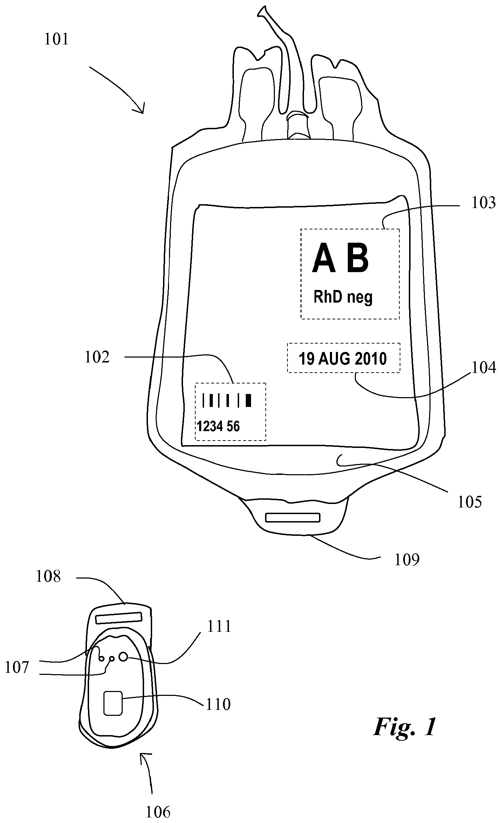

In the following, a number of different embodiments of a system for handling medical packages according to the invention will be described in detail with reference to the appended Figures 1-5.

Figure 1 shows a schematic illustration of a medical package 101 in the form of a blood bag. The package 101 is handled in a system with a plurality of, figuratively speaking, similar package units and is therefore at its outer surface provided with a label with information, which at least includes a unique identity code 102 and other relevant information. 103, 104, which identifies and/or characterizes a specific product content 105 in the package 101 in a unique, for the use of the specific product content, necessary way. In the case of package 101 as shown in Figure 1, the unique identity code 102 is a bar code that is intended to be read with a barcode scanner, combined with a bag number (corresponding bar code) that can be read visually by a system operator, to also allow for visual reading of the unique identity of the blood bag and input of it manually in the system if this for some reason is necessary. It should be noted that the information on the blood bag as shown in Figure 1 is not identical to the information that would appear on an actual blood bag. For the simplicity reason, the information in the drawing is incomplete and schematic and only illustrated as a support for the present description.

With continued reference to Figure 1, the system of the invention comprises at least one tracking device 106 arranged to accompany the package 101 with the specific product content 105 over a period of time that extends from the entry of the package 101 in the system to a possible decision on administration of the specific product content 105 to an intended recipient.

The tracking device 106 in the system according to the invention can be arranged to accompany the package 101 with the specific product content during the above time period in a number of different ways. In one embodiment of the invention, the tracking device 106 has a first fastening device 108 which is adapted to be attached to the second fastening device 109 of package 101, as shown in Figure 1. The tracking device 106 may advantageously be arranged to accompany the package 101 by attaching it to the package by means of a strap, string, a rope or a chain. Alternatively, the tracking device is arranged to accompany the package by attaching it using an adhesive on the exterior of the package, or packaged in a preferably transparent exterior package common to the package.

The tracking device 106 in the system of the invention includes an integrated data logger/data storage device (not shown in the figures) which is arranged to, during above time period, record data about at least one environmental parameter that affects the specific product content's 105 remaining lifetime. The ambient parameters, which using an appropriate sensor device is logged continuously or at least regularly at frequent intervals, by the data logger are preferably at least the temperature of the environment where the package is. In such a case, the tracking device 106 includes one for this purpose suitable temperature sensor. The data logger can also be arranged to record data about several environmental parameters simultaneously, such as both temperature and moisture content, or to register any other parameter that is related to the environment, such as light conditions, and/or radiation, such as radioactive radiation, by sensing using one or more sensor devices.

To enable the continuous or regular data logging of ambient data, the tracking device 106 comprises, in a preferred embodiment, an integrated power source (not shown in the drawings) with a capacity that is selected to ensure the data logger power supply throughout the aforementioned period. The power source can be of any suitable type whatsoever, but preferably is provided in the form of a battery, a fuel cell, or a capacitive energy source, such as a super capacitance. In one version, the battery or capacitance may be charged inductively.

The tracking device 106 according to the invention also includes a calculation function, including for example a calculation algorithm, which based on the logged data and time calculates a value for the remaining lifetime, for example, an estimated number of days remaining lifetime or a calculated expiration date of the product content that tracking device accompanies. Calculation algorithm has different form depending on which ambient parameter(s) that is/are logged, but primarily is based on prior knowledge about the durability of the type of product content available in the current package in relation to the environment parameter(s) logged by the data logger in the current tracking device. In the system according to the invention may therefore include individual tracking devices with different types of data logging and various algorithms if the system is designed to handle different types of package products whose contents are different in terms of storage characteristics. The system, which only will handle packages with product content of one main type (such as bags with transfusion blood) one could of course program the necessary computational algorithms in the tracking device from the start, but even in such cases it is advantageous for the system allowing entering new computational algorithms in the system's tracking device if this is necessary for some reason.

The tracking device 106 or tracking devices in the system according to the invention also includes an integrated indicator device 107, which is arranged to indicate to an operator, at least if the specific product content 105 in a package have a remaining lifetime or not. Advantageously, the integrated indicator device includes, as shown schematically in Figure 1, one or more light emitting diodes (LEDs) 107 and/or a sound generator (not shown in the figures) that show or indicate to an operator whether the product content has remaining lifetime or not. Such an indicator device increases security of the system as the packages with the indicated completed useful lifetime of the product content can immediately be removed from the handling of the system by an operator for special control and discarded if it is necessary.

The integrated indicator device of the tracking device 106 may additionally or alternatively include a display 110, as indicated schematically in Figure 1, primarily for the displaying an estimated remaining useful lifetime and/or an expiry date for the specific product content 105 in the current package. Such a display or indicator device is advantageous because the system operator can easily see the estimated remaining lifetime of each package and therefore use this information, e.g. when placing several packages in a cold room, or when choosing a specific package for administration to a recipient.

In one embodiment, where the tracking device 106 is arranged to accompany a package 101 containing a blood product 105, the integrated indicator device 110 is arranged to show a rejection indication if it has been exposed to an ambient temperature with a maximum and minimum value, so that this particular package can immediately be removed from the system and discarded by an operator when he or she becomes aware of cassation indication.

In one embodiment, the tracking device is configured with temperature ranges and can thus sett which maximum/minimum temperature is required depending on the package content, region, demands, etc.

For example, in an application in which the package contains blood, the temperature range which is default in the tracking device can be according to Table 1:

**Table 1**

| **Temperature** | **Hours remaining for which the blood is valid** |
|---|---|
| <1 °C | 0 hours |
| <10 ° C | 1008 hours |
| <24 ° C | 24 hours |
| <30 ° C | 4 hours |
| > = 30 ° C | 0 hours |

The table shows in the left column values for blood bags ambient temperature and the time left for which the blood is valid, in the right column.

Each tracking unit 106 of the system according to the invention has a unique device identity (not shown in the figures) that can be read and in the system it is connected with the unique identity code 102 and other relevant information, 103, 104 on the package 101 with which the tracking device accompanies, to ensure that all relevant data relating to the package-specific product content 105, both data input by the operator and the data generated by the data logger in the tracking device 106 or other system devices in the system, can be handled and transferred between the system devices without risk of confusion.

In one preferred embodiment of the system according to the invention, the unique device identity of the tracking device 106 comprises a visible marking (not shown) which is arranged to be read visually by an operator. Such a visually readable marking on the tracking device, such as a unit number, provide especially when entering new products into the system, a higher level of security because a system operator can visually compare the label on the tracking device with an equally visually readable identity code of a package that is fed into the system for the first time. Such visually readable markings or data on the tracking devices and the package also provide an opportunity for cross-checking and thus greater safety for the continued management of package that is already entered in the system.

In one embodiment of the invention, the unique device identity of the tracking device 106 is also arranged to be read from an optical marking, such as a bar code, using an optical reader. The unique device identity of the tracking device 106 can alternatively or additionally be arranged to be read wirelessly via radio signals, infrared or capacitively. Preferably, the tracking device 106 includes an antenna for wireless transmission of data via radio waves or infrared or capacitive transmission. A capacitive readout of data can be achieved by two "transmitting" electrode plates, which are arranged inside the tracking unit's outer casing, brought close to two "receiving" electrode plates of another read system unit in the system of the invention.

In a particularly advantageous embodiment of the system according to the invention, the tracking device 106 is arranged for wireless transmission of data via radio waves over one or more of the frequency bands 900 MHz, 2.4 GHz, 5.2 GHz and/or 5.8 GHz. Radio communication over these bands is free worldwide and frequencies are also allowed to be use in hospitals.

Advantageously, the tracking device 106 includes a communication device 111 for receiving signals from an operator, e.g. for the reception of a signal from the operator to activate the display of any remaining lifetime of the tracking device's LEDs 107 or a display of a value of the estimated remaining lifetime on a display 110. The communication device can favorably be provided in the form of a capacitive or mechanical switch 111 and for example be in form of touch buttons, such as one or more foil buttons. The tracking device 106 may alternatively include a communication device in the form of a piezoelectric-crystal reacting to knock or motion. Such a communication device allows an operator to communicate with the tracking device, e.g. by knocking it into a table top, shake it, or the like.

The tracking device is preferably provided with an outer shell with no cracks, folds, grooves or moving parts to facilitate cleaning, and also for reuse of the tracking device in the system after reprogramming. The previously mentioned LEDs 107 are preferably arranged in translucent parts of the outer casing. The outer casing is preferably made of one or more suitable plastic material and has no metallic surfaces as these are difficult to keep clean and may corrode when cleaning and also could complicate passage of radio waves. In a particularly advantageous embodiment, the tracking device 106 has a fluid-tight casing, whereby components included in the tracking device have a design and made of materials that permit washing, preferably including centrifugation, in a conventional washing machine with a water based wash agent at a temperature above 70°C. This embodiment makes it possible to clean the tracking device in conventional, often existing equipment, and then be able to reuse the tracking device after a reprogramming of the same and updating its data into the computer system that keeps track of packages and their accompanying tracking devices.

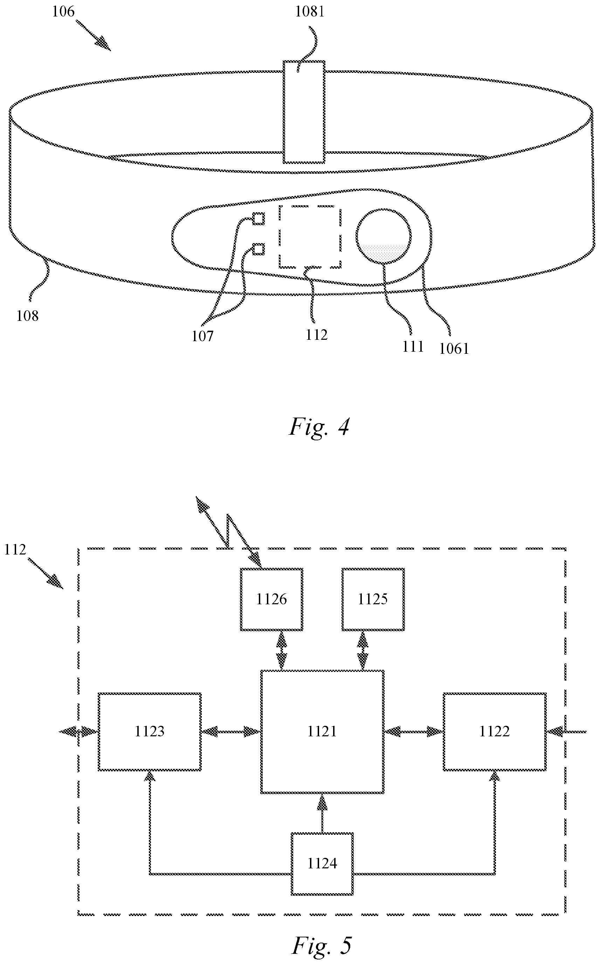

Figure 4 illustrates a second embodiment of the tracking device 106, including a housing 1061, indicating means 107, attaching device 108 and communications parts 111.

The outer casing 1061 of the housing is arranged with no crevices, folds, grooves or moving parts to facilitate cleaning, and that after reprogramming to reuse tracking device in the system. Indicating means107 can be LEDs 107 with different colors and can be arranged under translucent parts of the outer casing. The outer casing is preferably made of one or more suitable plastic materials. Even here, the tracking device 106 has a fluid-tight casing, whereby the components of the tracking device have a design and made of material that allow cleaning. Attachment parts 108 are arranged as a band that can enclose the package. The band can be fitted with a locking device 1081 that may also allow modification of the strap length adjustment for different package sizes. The communication device 111 is intended for the reception of signals from a user or operator to activate the display for any remaining lifetime with the tracking device's LEDs 107 or a display for a value of the estimated remaining lifetime on a display (not shown). The communication device may be in the form of a capacitive or mechanical button or switch in the outer casing. Even this embodiment of the tracking device 106 may include a communication device in the form of a piezoelectric-crystal reacting to knock.

112 designates the electronics of the tracking device. One or more energy sources can be integrated with the electronics. Figure 5 shows a schematic diagram of the electronics 112 The electronics may include a microprocessor 1121, one or more sensors 1122, interface 1123 and an energy source 1124, a memory 1125 and the communications unit 1126. The microprocessor 1121 is configured to, with respect to instructions stored in memory 1125 or an internal memory, control the tracking device's functions, such as reading sensors, execute the measurement program, or indicate a fault or alarm.

The sensor 1122 detects the parameters needed, such as temperature, radiation, humidity, etc. The interface 1123 is arranged to read the incoming instructions, e.g. from the button 111 and operate the indicator system. The memory 1125 may include instructions to the processor and/or store data. The communication unit 1126 is arranged to communicate with the outside world, wireless or wired.

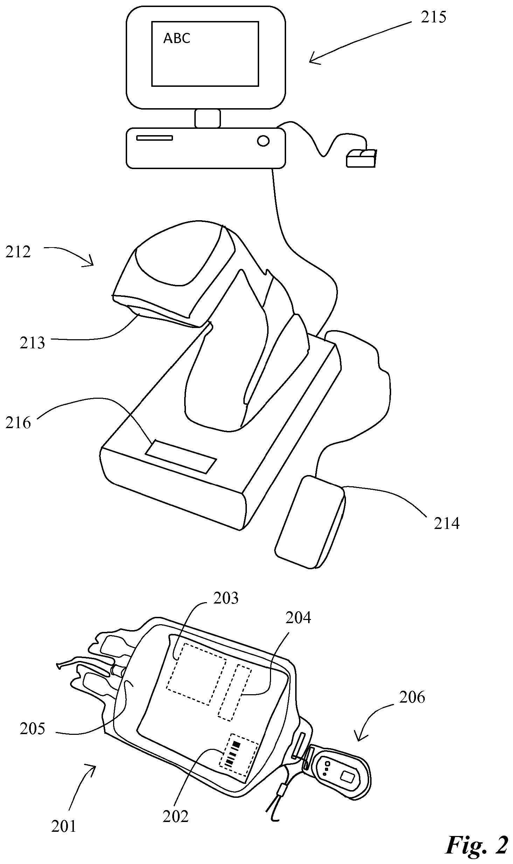

In a particularly preferred embodiment of the system according to the invention, illustrated schematically in Figure 2, the aforementioned other system devices in the system comprise at least one access point 212 which, for the entry of new medical package in the system, is provided with means for reading the unique identity code 202 and the other relevant information, 203, 204 on a specific package 201 and means for transmitting the identity code and information to at least one data record in a database included in the system, and also means for reading the unique device identity of a tracking device 206 which will accompany the specific package 201 in the continued management of the system and means for transmission of this unique device identity to the database to be linked with the above data item or data items.

In another particularly preferred embodiment of the system according to the invention, the above-mentioned other system devices in the system comprise at least one access point 212 which, for the handling of medical package that is already entered into the system without risk of confusion and updating of data on package and location of the system, is provided with means for reading the unique device identity of each tracking device 206 included a specific package whose previous data already exists at least as one data record in the aforementioned database before the package 201 can continue in the system, past the access point.

In another particularly preferred embodiment of the system according to the invention, the above-mentioned other system devices in the system comprise at least one access point 212 which for the retrieval and updating of data on at least one environmental parameter that affects the remaining lifetime of a specific package product content, is provided with means for reading any new data relating to the at least one environmental parameter recorded by the data logger with each tracking device 206 accompanying a specific package, whose previous data already exists as last one data record in the aforementioned database, when this specific package 201 passes the access point 212.

It should be noted that the above three functions, which can be performed by an access point may be provided separately by different access points that are located in different parts of the system, and that there may be access points in the system which are equipped with several features, or can perform all three functions.

Each access point 212 in the system according to the invention preferably comprises at least one optical scanner 213, preferably a laser scanner for barcode or quick response code or a camera, for reading the unique identifier 202 and other relevant information, 203, 204 on a specific packing 201.

Each access point 212 in the system according to the invention preferably also includes at least one wireless reader 214 for radio signals, infrared, or capacitive readout of the unique device identity of a tracking device 206 which will be supplied with or accompanying a specific packing two hundred and first

Access point 212 in the system according to the invention can also with advantage include at least one wireless reader 214 for radio signals, infrared, or capacitive readout of that data on at least one ambient parameter recorded by the logger with a tracking device 206 which accompanies a specific package 201. This embodiment makes it possible to read the current estimated remaining lifetime of the product content of that specific package each time the package passes such an access point, thereby allowing retrieval of latest estimated remaining lifetime as possible to a central computer system.

The access point 212 in the system according to the invention can with advantage include at least one communication link to a server, preferably of type LAN (Ethernet), wireless LAN, GSM or GPRS, for transmitting data between the access point 212 and the server and/or other system devices in the system.

The access point 212 in the system according to the invention can with advantage include at least one (local) computer device 215 arranged to handle reading, processing and communication of information in the system, and communication with an operator.

Each access point 212 in the system according to the invention preferably comprises at least one indicator device for communication with an operator, preferably including LED/illumine surfaces, a display 216 and/or a sound generator. The access point 212 in the system according to the invention can also with advantage include at least one communication means (not shown in the drawings), preferably in form of a capacitive or mechanical key or an piezoelectric-crystal reacting to knock, which can be affected by an operator.

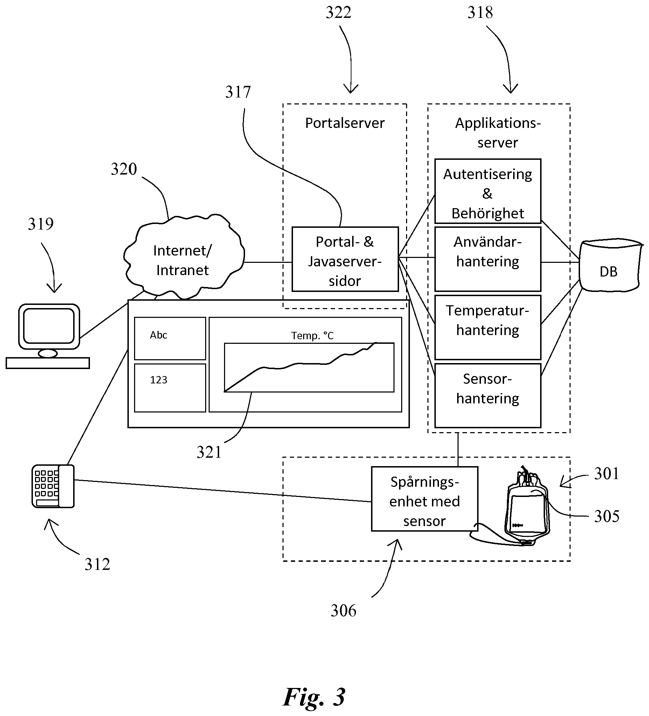

In one preferred embodiment of the system, according to the invention, schematically illustrated in Figure 3, the aforementioned other system devices in the system comprise at least one portal 317 for system users who can communicate with at least one data server 318 that stores data relating to all medical packages that are entered into the system. Provision of such an access point 317 enables the system users to access these data from a system user selectable location. The data stored in the data server in this embodiment includes at least: a unique identifier for each specific package 301 inserted into the system, a unique device identity for the specific detection unit 306 that comes with this specific package in the management of the system, a latest reading date of the information concerning the specific package 301 stored in the data server 318, a specification of the last access point 312 as the specific detection unit 306 and thus the specific package 301 has passed through the system, an estimated expiration date for a specific product content 305 in the specific package 301, and other relevant information that identifies and characterizes the specific product content 305 in a unique, for the use of the specific product content necessary way, which data the system users may access from at least one user terminal 319 which is connected to the portal 317 for system users. The system according to this preferred embodiment allows system users to be able to find a specific package or a specific product content as requested, to follow this specific package or this specific product content movement and current location in the system, and to be able to consult a calculated expiration date for the specific product content of a specific package.

The portal 317 for system users includes preferably at least a search function, which is provided to permit search for packages 301 in the system containing the specific product content 305 and the estimated remaining lifetime of a specific user needs, as well as search for specific packages 301 that are physically located close to the specific user. The search function is preferably accessible from a terminal 319 via the Internet 320 or intranet.

Preferably, the portal 317 for system users also allows access to a detailed storage history of a specific package, preferably a temperature history 321, obtained from the ambient parameter data recorded by the integrated data logger in the tracking device 306 during the period of time the tracking device is shipped with the specific package 301 when handled in the system.

The data for medical package 301 that can be accessed via the portal 317 for system users can be saved in separate data records stored in multiple databases and/or servers 318, 322 and/or computers, whereby these separate data records are linked in the system to give the system user, user-friendly access to all relevant data concerning a specific product content 305 in a specific package 301 without risk of confusion.

The enclosed hardware and software in the system can with advantage be particularly fitted for handling packages containing a specific product, such as blood products 301, or for handling vaccine package.

The system according to the invention comprises, in a preferred embodiment, a number of access points that are located at least at the exit of a unit that makes or fills medical package with specific product content, at the entrance to a depot or an intermediate storage where the packages are stored, at the exit of the depot, or intermediate storage, and at the entrance to a treatment or care facility in which the administration of the specific product content is done.

The system according to the invention can with advantage include at least one access point in a transport vehicle designed to transport packages from a manufacturing or filling unit to a terminal or an intermediate storage, or from the depot or from the storage to a treatment or care facility, from the treatment or care facility to another treatment or care facility, from the treatment or care facility back to the depot or the intermediate storage, or from this treatment or care facility to another depot or another intermediate storage. The system according to the invention may thus comprise at least one access point on board a car, an ambulance, a truck, a trailer truck, an airplane, a helicopter, a boat or a ship, or a portable access point that can be transported to a desired position by an operator. One or more portable access points can with advantage used in the establishment of a system according to the invention of a disaster or war situations.

In the above, a number of different embodiments of the invention have been described with reference to the illustrations on the attached drawings. It must be understood that the described embodiments and details of the drawings alone should be seen as an example and that number of other embodiments of the invention are possible in the context of the attached patent claims.

Within the scope of the invention are also embodiments in which the system includes devices for detection, reading and/or logging of possible contamination of package, for example, in an embodiment in which at least one access point in the system has a specially designed scanner that uses laser scans of a specific package or tracking device reflectance and/or transmission within a particular wavelength range before the package with its accompanying tracking device is allowed to pass the access point. This embodiment of the system according to the invention allows that the packages discovered to be contaminated, can be removed from the system and discarded.

## Claims

1. System for handling medical packages, in which each individual package (101) is provided with at least one unique identity code (102) and other relevant information (103, 104) comprising at least one specification (103) about the packaged product content and packaging date (104), whereby the system comprises:
• at least one tracking device (106) which is arranged to accompany the package (101) with said specific product content over a period of time, which extends from the entry of said package (101) in the system to a possible decision on administration of that specific product content (105) to an intended recipient, whereby the tracking device (106) includes an integrated data logger, which is arranged to record logged data and time during said period of time about at least one environmental parameter that affects lifetime of said specific product content (105), at least one means for reading said unique identity code (102), the tracking device (106) includes a calculation function, which based on the logged data and time calculates a value for said remaining lifetime,
the tracking device (106) comprises:
• an integrated indication device (107), which is arranged to indicate, at least about the specific product content (105), remaining lifetime,
• a communication device (111) for receiving a signal to activate indication of any remaining lifetime with said indication device (107),
the tracking device (106) has a unique device identity that is readable and in the system associated to said unique identity code (102) and said other relevant information (103, 104) on said package (101), to ensure that data relating to the specific product content (105), both input data and data generated by the data logger of the tracking device (106) or by additional devices in the system, can be handled and transferred between said system units, and
whereby the system comprises at least one means for reading said unique device identity of the tracking device (106),
**characterized in that** the tracking device (106) comprises a fluid-tight casing, and that the components of the tracking device are designed and made of materials that allow cleaning in a conventional washing machine with a water based wash agent at a temperature above 70°C.

2. System according to claim 1, **characterized in that** the tracking device (106) is arranged to accompany said package (101) with the specific product content during the same period of time by: tracking device comprises a first fastening device (108) adapted to be attached to a second fastening device (109) of the package and/or attached together with the package with help of a strap, a band, a rope or a chain and/or attached by means of an adhesive on exterior of the package and/or packaged in common outer packaging.

3. System according to any of the preceding claims,
**characterized in that** the tracking device (106) further comprises one or several of:
• at least one sensor device arranged for sensing ambient temperature, moisture content and/or light conditions and/or radiation, such that one or more of said parameters can be recorded by said data logger;
• an integrated power supply with a capacity selected to power the data logger throughout said period of time or an integrated power source in form of a battery, a fuel cell, or a capacitor;
• one or more of the light emitting diodes (107) and/or a sound generator and/or a display (110), primarily for reproducing the estimated remaining lifetime of the specific product content (105);
• a communication portion in form of one or more of capacitive, mechanical switch (111) or a piezoelectric-crystal, which reacts to movement; or
• a visible mark arranged to be read visually.

4. System according to any of the preceding claims,
**characterized in that** the tracking device (106) is arranged to accompany a package (101) containing a blood product (105), and that the integrated indicator device (110) is arranged to show a cassation indication if the product has been exposed to an ambient temperature between a maximum and a min value.

5. System according to any of the preceding claims,
**characterized in that** the unique device identity of the tracking device (106) is arranged to read by an optical device, such as a bar code, using an optical reader and/or wirelessly via radio signals, infrared or capacitively.

6. System according to any of claims 1-5,
**characterized in that** said other system devices in the system includes at least one access point (212), which for the entry of new medical package in the system is equipped with means for reading said unique identity code (202) and that other relevant information (203, 204) on a specific package (201) and means for transmitting said identifier and information to at least one data record in a database included in the system, and means for reading said unique device identity of a tracking device (206) that will accompany the specific package (201) for the continued handling of the system and means for transmitting said unique device identity to said database for association to said data record or data records, and/or
at least one access point (212) which, for the handling of medical package that already is entered into the system and to update the data on said package and position in the system, is equipped with means for reading said unique device identity of each tracking device (206) accompanying a specific package whose previous data already exists as at least one data record in said database before said package (201) is passed on in the system past said access point (212), and /or
at least one access point (212) which, for the retrieval and updating of data about at least one environmental parameter that affects the remaining lifetime of a specific package product content, is provided with means for reading any new data on said at least one environmental parameter recorded by the data logger at each tracking device (206) accompanying a specific package, whose previous data already exists as at least one data record in said database when said specific package (201) passes said access point (212).

7. System according to claim 6,
**characterized in that** the access point (212) comprises one or more of:
• at least one optical reader (213), preferably a laser scanner for barcode or quick access code or a camera, for reading said unique identity code (202) and that other relevant information (203, 204) on a specific package (201);
• at least one wireless reader (214) for radio signals, infrared, or capacitive readout of said unique device identity of a tracking device (206), which accompanies a specific package (201);
• at least one wireless reader (214) for radio signals, infrared, or capacitive readout of the data about at least one environmental parameter recorded by the logger with a tracking device (206) provided with a specific package (201);
• at least one communication link to a server, preferably LAN (Ethernet), wireless LAN, GSM or GPRS, for transmitting data between said access point (212) and server and/or other system devices to the system;
• at least one computer device (215) arranged to handle reading, processing and communication of information in the system, and communication with an operator;
• at least one indication device for communication with an operator, preferably comprising light emitting diode/light panel, a display (216) and/or a sound generator;
• at least one communication device, preferably in form of a capacitive or mechanical key or an piezoelectric-crystal that responds to tapping, which can be affected by an operator.

8. System according to any of the preceding claims,
**characterized in that** said other system devices in the system include at least one portal (317) for system users who can communicate with at least one data server (318) that stores data relating to all medical package that is entered into the system, whereby said data stored in said data server at least comprises:
- a unique identifier for each specific package (301) which is inserted into the system,
- a unique device identity for the specific detection unit (306) that accompanies the specific package in the handling of the system,
- a latest read date of the information concerning the specific package (301) stored in said data server (318),
- a specification of the last access point (312), which said specific tracking device (306) and thus the specific packing (301) has passed into the system,
- an estimated expiration date for a specific product content (305) of the specific package (301), and
- other relevant information that identifies and characterizes the specific product content (305) in a unique, for the use of that specific product content necessary means, which data the system users may access from at least one user terminal (319) connected to said portal (317) for system users.

9. System according to any of the preceding claims,
**characterized in that** the system comprises a number of access points that are located at least:
- at an exit of a device that makes or fills medical package with product-specific content,
- at an entrance to a depot or an intermediate storage where said packages are stored,
- at an exit from the said depot or the store, and
- at an entrance to a treatment or care facility, in which the administration of the specific product content is carried out.

10. System according to any of the preceding claims,
**characterized in that** the system comprises at least one access point for use in a transport vehicle designed to transport packages from a manufacturing or filling unit to a depot or an intermediate storage, or from said depot or the store to a treatment or health care unit, from said treatment or care facility to another treatment or care facility, from the treatment or care facility back to the depot or store, or from that treatment or care facility to another depot or another intermediate storage.

11. System according to any of the preceding claims,
**characterized in that** the system comprises at least one access point for use on board a car, an ambulance, a truck, a trailer truck, an airplane, a helicopter, a boat or a ship, or a portable access point that can be carried to a desired position by an operator.

12. A tracking device for use in a system for handling medical packages, in which system each individual package (101) is provided with at least one unique identity code (102) and information (103, 104) comprising at least one specification (103) about the packaged product content and packaging date (104), the system further comprising at least one means for reading said unique identity code (102), wherein the tracking device is arranged to accompany the package (101) with said specific product content over a period of time, which extends from the entry of said package (101) in the system to a possible decision on administration of that specific product content (105) to an intended recipient, the tracking device (106) comprising:
• an integrated data logger, which is arranged to record logged data and time during said period of time about at least one environmental parameter that affects lifetime of said specific product content (105),
• a calculation function, which based on the logged data and time calculates a value for said remaining lifetime,
• an integrated indication device (107), which is arranged to indicate, at least about the specific product content (105), remaining lifetime,
• a communication device (111) for receiving a signal to activate indication of any remaining lifetime with said indication device (107),
• a unique device identity,
wherein said unique device identity is readable by the system and in the system associated to said unique identity code (102) and said other relevant information (103, 104) on said package (101), to ensure that data relating to the specific product content (105),
wherein both input data and data generated by the data logger of the tracking device (106) or by additional devices in the system, can be handled and transferred between said system units, and
whereby the system comprises at least one means for reading said unique device identity of the tracking device (106),
**characterized in that** the tracking device (106) further comprises a fluid-tight casing, and that the components of the tracking device are designed and made of materials that allow cleaning in a conventional washing machine with a water based wash agent at a temperature above 70°C.

13. A method for managing medical packages, whereby each individual package (101) is provided with at least one unique identity code (102) and other relevant information (103, 104), comprising at least one specification (103) of the packages product content and packaging date (104), which identifies and characterizes a specific product content (105) of the said package, whereby the method comprise providing the package with at least one tracking device (106), which is arranged to accompany said package (101) with the specific product content over a period of time ranging all the way from the entry of said package (101) to a possible decision on administration of the specific product content (105) to an intended recipient, wherein the tracking device (106) includes an integrated data logger, which is arranged to record logged data and time on at least one environmental parameter affecting that specific product content (105) lifetime during the same period of time, and the tracking device (106) comprises a fluid-tight casing, and that the components of the tracking device are designed and made of materials that allow cleaning in a conventional washing machine with a water based wash agent at a temperature above 70°C, the method further comprises the steps of:
- calculating a value for said remaining lifetime by the tracking device (106) and based on the logged data and time,
- indicating by the indicator device (107) of the tracking device (106) at least about the specific product content (105) remaining lifetime,
- enabling detection of any remaining lifetime by means of said indicator device (107),
- handling and transmitting data relating to the specific product content (105), both input data and data generated by the data logger of that tracking device (106) between that system is called, has a unique device identity that is readable and associate with that unique identifier (102) and that other relevant information (103, 104) of said package (101).

## Patentansprüche

1. System zur Handhabung medizinischer Verpackungen, wobei jede einzelne Verpackung (101) mit mindestens einem einzigartigen Identitätscode (102) und anderen relevanten Informationen (103, 104) versehen ist, die mindestens eine Beschreibung (103) über den Inhalt des verpackten Produkts und das Verpackungsdatum (104) umfassen, wobei das System umfasst:
• mindestens eine Verfolgungsvorrichtung (106), die so eingerichtet ist, dass sie die Verpackung (101) mit dem spezifischen Produktinhalt über einen Zeitraum begleitet, der sich von dem Eingang der Verpackung (101) in das System bis zu einer möglichen Entscheidung über die Verabreichung dieses spezifischen Produktinhalts (105) an einen vorgesehenen Empfänger erstreckt, wobei die Verfolgungsvorrichtung (106) einen integrierten Datenlogger beinhaltet, der so eingerichtet ist, dass er protokollierte Daten und Zeit während des Zeitraums über mindestens einen Umgebungsparameter aufzeichnet, der die Lebensdauer des spezifischen Produktinhalts (105) beeinflusst, mindestens ein Mittel zum Lesen des einzigartigen Identitätscodes (102), wobei die Verfolgungsvorrichtung (106) eine Berechnungsfunktion beinhaltet, die auf der Grundlage der protokollierten Daten und Zeit einen Wert für die Restlebensdauer berechnet,
die Verfolgungsvorrichtung (106) umfasst:
• eine integrierte Anzeigevorrichtung (107), die so eingerichtet ist, dass sie mindestens über den spezifischen Produktinhalt (105) die Restlebensdauer anzeigt,
• eine Kommunikationsvorrichtung (111) zum Empfangen eines Signals zum Aktivieren der Anzeige einer Restlebensdauer mit der Anzeigevorrichtung (107),
die Verfolgungsvorrichtung (106) eine einzigartige Vorrichtungsidentität aufweist, die lesbar ist und in dem System dem einzigartigen Identitätscode (102) und den anderen relevanten Informationen (103, 104) auf der Verpackung (101) zugeordnet ist, um sicherzustellen, dass Daten, die sich auf den spezifischen Produktinhalt (105) beziehen, sowohl Eingabedaten als auch Daten, die durch den Datenlogger der Verfolgungsvorrichtung (106) oder durch zusätzliche Vorrichtungen in dem System erzeugt werden, gehandhabt und zwischen den Systemeinheiten übertragen werden können, und
wobei das System mindestens ein Mittel zum Lesen der einzigartigen Vorrichtungsidentität der Verfolgungsvorrichtung (106) umfasst,
**dadurch gekennzeichnet, dass** die Verfolgungsvorrichtung (106) ein fluiddichtes Gehäuse umfasst, und dass die Komponenten der Verfolgungsvorrichtung aus Materialien konstruiert und hergestellt sind, die eine Reinigung in einer herkömmlichen Waschmaschine mit einem Waschmittel auf Wasserbasis bei einer Temperatur über 70°C ermöglichen.

2. System nach Anspruch 1,
**dadurch gekennzeichnet, dass** die Verfolgungsvorrichtung (106) so eingerichtet ist, dass sie die Verpackung (101) mit dem spezifischen Produktinhalt während des gleichen Zeitraums begleitet, indem: die Verfolgungsvorrichtung eine erste Befestigungsvorrichtung (108) umfasst, die geeignet ist, an einer zweiten Befestigungsvorrichtung (109) der Verpackung befestigt zu werden und/oder mit Hilfe eines Riemens, eines Bandes, eines Seils oder einer Kette mit der Verpackung zusammen befestigt zu werden und/oder mit Hilfe eines Klebstoffs an der Außenseite der Verpackung befestigt und/oder in einer gemeinsamen Außenverpackung verpackt zu werden.

3. System nach einem der vorstehenden Ansprüche,
**dadurch gekennzeichnet, dass** die Verfolgungsvorrichtung (106) weiter eine oder mehrere umfasst von:
• mindestens einer Sensorvorrichtung, die so eingerichtet ist, dass sie die Umgebungstemperatur, den Feuchtigkeitsgehalt und/oder die Lichtverhältnisse und/oder die Strahlung erfasst, so dass einer oder mehrere dieser Parameter durch den Datenlogger aufgezeichnet werden können;
• einer integrierten Stromversorgung mit einem Leistungsvermögen, das so gewählt ist, dass es den Datenlogger während des gesamten Zeitraums versorgt, oder einer integrierten Stromquelle in Form einer Batterie, einer Brennstoffzelle oder einem Kondensator;
• einer oder mehreren der lichtemittierenden Dioden (107) und/oder einem Tongenerator und/oder einer Anzeige (110), hauptsächlich zur Wiedergabe der geschätzten Restlebensdauer des spezifischen Produktinhalts (105);
• einem Kommunikationsabschnitt in Form eines oder mehrerer kapazitiver, mechanischer Schalter (111) oder eines piezoelektrischen Kristalls, der auf Bewegung reagiert; oder
• einer sichtbaren Markierung, die so eingerichtet ist, dass sie visuell gelesen werden kann.

4. System nach einem der vorstehenden Ansprüche,
**dadurch gekennzeichnet, dass** die Verfolgungsvorrichtung (106) so eingerichtet ist, dass sie eine Verpackung (101) begleitet, die ein Blutprodukt (105) enthält, und dass die integrierte Anzeigevorrichtung (110) so eingerichtet ist, dass sie eine Kassationsanzeige anzeigt, wenn das Produkt einer Umgebungstemperatur zwischen einem Maximal- und einem Minimalwert ausgesetzt war.

5. System nach einem der vorstehenden Ansprüche,
**dadurch gekennzeichnet, dass** die einzigartige Vorrichtungsidentität der Verfolgungsvorrichtung (106) so eingerichtet ist, dass sie von einer optischen Vorrichtung, wie einem Strichcode, unter Verwendung eines optischen Lesegeräts und/oder drahtlos über Funksignale, Infrarot oder kapazitiv gelesen wird.

6. System nach einem der Ansprüche 1-5,
**dadurch gekennzeichnet, dass** die anderen Systemvorrichtungen in dem System mindestens einen Zugangspunkt (212) beinhalten, der für den Eingang einer neuen medizinischen Verpackung in das System mit Mitteln zum Lesen des einzigartigen Identitätscodes (202) und anderer relevanter Informationen (203, 204) über eine spezifische Verpackung (201) und Mitteln zum Übertragen der Kennung und der Informationen zu mindestens einem Datensatz in einer in dem System enthaltenen Datenbank ausgestattet ist, und Mittel zum Lesen der einzigartigen Vorrichtungsidentität einer Verfolgungsvorrichtung (206), die die spezifische Verpackung (201) für die weitere Handhabung des Systems begleiten wird, und Mittel zum Übertragen der einzigartigen Vorrichtungsidentität an die Datenbank zur Zuordnung zu dem Datensatz oder den Datensätzen, und/oder
mindestens einen Zugangspunkt (212), der für die Handhabung einer bereits in das System eingegebenen medizinischen Verpackung und zur Aktualisierung der Daten über diese Verpackung und Position im System mit Mitteln zum Lesen der einzigartigen Vorrichtungsidentität jeder Verfolgungsvorrichtung (206) ausgestattet ist, die eine spezifische Verpackung begleitet, deren vorherige Daten bereits als mindestens ein Datensatz in der Datenbank vorhanden sind, bevor die Verpackung (201) im System über den Zugangspunkt (212) hinaus weitergeleitet wird, und/oder
mindestens einen Zugangspunkt (212), der für das Abrufen und Aktualisieren von Daten über mindestens einen Umgebungsparameter, der die Restlebensdauer eines spezifischen Verpackungsproduktinhalts beeinflusst, mit Mitteln zum Lesen aller neuen Daten über den mindestens einen Umgebungsparameter versehen ist, die von dem Datenlogger an jeder Verfolgungsvorrichtung (206) aufgezeichnet werden, die eine spezifische Verpackung begleitet, deren vorherige Daten bereits als mindestens ein Datensatz in der Datenbank existieren, wenn die spezifische Verpackung (201) den Zugangspunkt (212) passiert.

7. System nach Anspruch 6,
**dadurch gekennzeichnet, dass** der Zugangspunkt (212) einen oder mehrere umfasst von:
• mindestens einem optischen Leser (213), vorzugsweise einen Laserscanner für Strichcode oder Schnellzugangscode oder eine Kamera, zum Lesen des einzigartigen Identitätscodes (202) und der anderen relevanten Informationen (203, 204) auf einer spezifischen Verpackung (201);
• mindestens einem drahtlosen Lesegerät (214) für Funksignale, Infrarot oder kapazitives Auslesen der einzigartigen Vorrichtungsidentität einer Verfolgungsvorrichtung (206), die eine spezifische Verpackung (201) begleitet;
• mindestens einem drahtlosen Lesegerät (214) für Funksignale, Infrarot oder kapazitives Auslesen der Daten über mindestens einen Umgebungsparameter, die von dem Logger mit einer mit einer spezifischen Verpackung (201) versehenen Verfolgungsvorrichtung (206) aufgezeichnet wurden;
• mindestens einer Kommunikationsverbindung zu einem Server, vorzugsweise LAN (Ethernet), drahtloses LAN, GSM oder GPRS, zur Übertragung von Daten zwischen dem Zugangspunkt (212) und dem Server und/oder anderen Systemvorrichtungen zu dem System;
• mindestens einer Computervorrichtung (215), die eingerichtet ist, um das Lesen, Verarbeiten und Kommunizieren von Informationen im System und die Kommunikation mit einer Bedienungsperson zu handhaben;
• mindestens einer Anzeigevorrichtung zur Kommunikation mit einer Bedienungsperson, die vorzugsweise eine Leuchtdiode/Lichttafel, eine Anzeige (216) und/oder einen Tongenerator umfasst;
• mindestens einer Kommunikationsvorrichtung, vorzugsweise in Form einer kapazitiven oder mechanischen Taste oder eines piezoelektrischen Kristalls, die auf ein Klopfen reagiert, das von einer Bedienungsperson beeinflusst werden kann.

8. System nach einem der vorstehenden Ansprüche,
**dadurch gekennzeichnet, dass** die anderen Systemvorrichtungen in dem System mindestens ein Portal (317) für Systembenutzer beinhalten, die mit mindestens einem Datenserver (318) kommunizieren können, der Daten speichert, die sich auf die gesamte medizinische Verpackung beziehen, die in das System eingegeben wird, wobei die in dem Datenserver gespeicherten Daten mindestens umfassen:
- eine einzigartige Kennung für jede spezifische Verpackung (301), die in das System eingegeben wird,
- eine einzigartige Vorrichtungsidentität für die spezifische Erkennungseinheit (306), die der spezifischen Verpackung bei der Handhabung des Systems beiliegt,
- ein spätestes Lesedatum der Informationen über die spezifische Verpackung (301), die in dem Datenserver (318) gespeichert sind,
- eine Beschreibung des letzten Zugangspunktes (312), den die spezifische Verfolgungsvorrichtung (306) und damit die spezifische Verpackung (301) in dem System passiert hat,
- ein geschätztes Ablaufdatum für einen spezifischen Produktinhalt (305) der spezifischen Verpackung (301), und
- andere relevante Informationen, die den spezifischen Produktinhalt (305) in einer einzigartigen, für die Verwendung dieses spezifischen Produktinhalts erforderlichen Weise identifizieren und charakterisieren, auf welche Daten die Systembenutzer von mindestens einem mit dem Portal (317) verbundenen Benutzerterminal (319) für Systembenutzer zugreifen können.

9. System nach einem der vorstehenden Ansprüche,
**dadurch gekennzeichnet, dass** das System eine Anzahl von Zugangspunkten umfasst, die mindestens lokalisiert sind:
- an einem Ausgang einer Vorrichtung, die eine medizinische Verpackung mit produktspezifischem Inhalt herstellt oder füllt,
- an einem Eingang zu einem Depot oder einem Zwischenlager, in dem die Verpackungen gelagert werden,
- an einem Ausgang des genannten Depots oder des Lagers, und
- an einem Eingang zu einer Behandlungs- oder Pflegeeinrichtung, in der die Verabreichung des spezifischen Produktinhalts durchgeführt wird.

10. System nach einem der vorstehenden Ansprüche,
**dadurch gekennzeichnet, dass** das System mindestens einen Zugangspunkt zur Verwendung in einem Transportfahrzeug umfasst, das dafür ausgelegt ist, Verpackungen von einer Herstellungs- oder Abfülleinheit zu einem Depot oder einem Zwischenlager oder von dem Depot oder dem Lager zu einer Behandlungs- oder Gesundheitsversorgungseinheit, von der Behandlungs- oder Versorgungseinrichtung zu einer anderen Behandlungs- oder Versorgungseinrichtung, von der Behandlungs- oder Versorgungseinrichtung zurück zu dem Depot oder Lager oder von dieser Behandlungs- oder Versorgungseinrichtung zu einem anderen Depot oder einem anderen Zwischenlager zu transportieren.

11. System nach einem der vorstehenden Ansprüche,
**dadurch gekennzeichnet, dass** das System mindestens einen Zugangspunkt zur Verwendung an Bord eines Autos, eines Krankenwagens, eines Lastwagens, eines Anhängerlastwagens, eines Flugzeugs, eines Hubschraubers, eines Bootes oder eines Schiffes oder einen tragbaren Zugangspunkt umfasst, der von einer Bedienungsperson zu einer gewünschten Position befördert werden kann.

12. Verfolgungsvorrichtung zur Verwendung in einem System zur Handhabung medizinischer Verpackungen, wobei in dem System jede einzelne Verpackung (101) mit mindestens einem einzigartigen Identitätscode (102) und Informationen (103, 104) versehen ist, die mindestens eine Beschreibung (103) über den Inhalt des verpackten Produkts und das Verpackungsdatum (104) umfassen, wobei das System weiter mindestens ein Mittel zum Lesen des einzigartigen Identitätscodes (102) umfasst, wobei die Verfolgungsvorrichtung so eingerichtet ist, dass sie die Verpackung (101) mit dem spezifischen Produktinhalt über einen Zeitraum begleitet, der sich von dem Eingang der Verpackung (101) in das System bis zu einer möglichen Entscheidung über die Verabreichung dieses spezifischen Produktinhalts (105) an einen vorgesehenen Empfänger erstreckt, wobei die Verfolgungsvorrichtung (106) umfasst:
• einen integrierten Datenlogger, der so eingerichtet ist, dass er protokollierte Daten und Zeit während des Zeitraums über mindestens einen Umgebungsparameter aufzeichnet, der die Lebensdauer des spezifischen Produktinhalts (105) beeinflusst,
• eine Berechnungsfunktion, die auf der Grundlage der protokollierten Daten und der Zeit einen Wert für die Restlebensdauer berechnet,
• eine integrierte Anzeigevorrichtung (107), die so eingerichtet ist, dass sie mindestens über den spezifischen Produktinhalt (105) die Restlebensdauer anzeigt,
• eine Kommunikationsvorrichtung (111) zum Empfangen eines Signals zum Aktivieren der Anzeige einer Restlebensdauer mit der Anzeigevorrichtung (107),
• eine einzigartige Vorrichtungsidentität,
wobei die einzigartige Vorrichtungsidentität durch das System und in dem System lesbar ist, das dem einzigartigen Identitätscode (102) und den anderen relevanten Informationen (103, 104) auf der Verpackung (101) zugeordnet ist, um sicherzustellen, dass Daten sich auf den spezifischen Produktinhalt (105) beziehen,
wobei sowohl Eingabedaten als auch durch den Datenlogger der Verfolgungsvorrichtung (106) erzeugte Daten oder durch zusätzliche Vorrichtungen im System gehandhabt und zwischen den Systemeinheiten übertragen werden können, und
wobei das System mindestens ein Mittel zum Lesen der einzigartigen Vorrichtungsidentität der Verfolgungsvorrichtung (106) umfasst,
**dadurch gekennzeichnet, dass** die Verfolgungsvorrichtung (106) weiter ein fluiddichtes Gehäuse umfasst und dass die Komponenten der Verfolgungsvorrichtung aus Materialien konstruiert und hergestellt sind, die eine Reinigung in einer herkömmlichen Waschmaschine mit einem Waschmittel auf Wasserbasis bei einer Temperatur über 70°C ermöglichen.

13. Verfahren zum Verwalten von medizinischen Verpackungen, wobei jede einzelne Verpackung (101) mit mindestens einem einzigartigen Identitätscode (102) und anderen relevanten Informationen (103, 104) versehen wird, umfassend mindestens eine Beschreibung (103) des Produktinhalts der Verpackung und des Verpackungsdatums (104), die einen spezifischen Produktinhalt (105) der Verpackung identifiziert und charakterisiert, wobei das Verfahren das Versehen der Verpackung mit mindestens einer Verfolgungsvorrichtung (106) umfasst, die so eingerichtet ist, dass sie die Verpackung (101) mit dem spezifischen Produktinhalt über einen Zeitraum begleitet, der sich über den gesamten Weg von dem Eingang der Verpackung (101) bis zu einer möglichen Entscheidung über die Verabreichung des spezifischen Produktinhalts (105) an einen vorgesehenen Empfänger erstreckt, wobei die Verfolgungsvorrichtung (106) einen integrierten Datenlogger beinhaltet, der so eingerichtet ist, dass er protokollierte Daten und Zeit zu mindestens einem Umgebungsparameter aufzeichnet, der die Lebensdauer dieses spezifischen Produktinhalts (105) während des gleichen Zeitraums beeinflusst, und die Verfolgungsvorrichtung (106) ein fluiddichtes Gehäuse umfasst, und dass die Komponenten der Verfolgungsvorrichtung aus Materialien konstruiert und hergestellt sind, die eine Reinigung in einer herkömmlichen Waschmaschine mit einem Waschmittel auf Wasserbasis bei einer Temperatur über 70°C ermöglichen, wobei das Verfahren weiter die Schritte umfasst von:
- Berechnen eines Wertes für die Restlebensdauer durch die Verfolgungsvorrichtung (106) und basierend auf den aufgezeichneten Daten und der Zeit,
- Anzeigen durch die Anzeigevorrichtung (107) der Verfolgungsvorrichtung (106) mindestens über den spezifischen Produktinhalt (105) der Restlebensdauer,
- Ermöglichung der Feststellung einer etwaigen Restlebensdauer mit Hilfe der Anzeigevorrichtung (107),
- Handhabung und Übertragung von Daten, die sich auf den spezifischen Produktinhalt (105) beziehen, sowohl Eingabedaten als auch Daten, die durch den Datenlogger dieser Verfolgungsvorrichtung (106) zwischen diesem System erzeugt werden, eine einzigartige Vorrichtungsidentität haben, die lesbar ist und mit dieser einzigartigen Kennungsidentität (102) und diesen anderen relevanten Informationen (103, 104) der Verpackung (101) verbunden ist.

## Revendications

1. Système pour la gestion de conditionnements médicaux équipés, dans lequel chaque conditionnement individuel (101) est muni d'au moins un code d'identité unique (102) et d'autres informations pertinentes (103, 104), comprenant au moins une spécification (103) concernant le produit conditionné et la date de conditionnement (104), selon lequel le système comprend :
• au moins un dispositif de repérage (106) conçu pour accompagner ledit conditionnement (101) avec ledit contenu de produit spécifique sur une période de temps, qui s'étend de l'entrée dudit conditionnement (101) dans le système jusqu'à une décision éventuelle concernant l'administration du contenu de produit spécifique (105) à un destinataire prévu, le dispositif de repérage (106) incluant un enregistreur de données intégré, qui est conçu pour enregistrer les données et l'heure enregistrées pendant ladite période de temps concernant au moins un paramètre environnemental affectant la durée de vie dudit contenu de produit spécifique (105), au moins un moyen pour la lecture dudit code d'identité unique (102), le dispositif de repérage (106) comprenant une fonction de calcul qui, sur la base des données et de l'heure enregistrées, calcule une valeur pour ladite durée de vie restante,
le dispositif de repérage (106) comprend :
• un dispositif d'indication intégré (107), qui est conçu pour indiquer, au moins à propos du contenu de produit spécifique (105), la durée de vie restante,
• un dispositif de communication (111) pour la réception d'un signal pour activer l'indication de toute durée de vie restante avec ledit dispositif d'indication (107),
le dispositif de repérage (106) présente une identité de dispositif unique qui est lisible et, dans le système, associée audit code d'identité unique (102) et auxdites autres informations pertinentes (103, 104) sur ledit conditionnement (101), pour garantir que les données relatives au contenu de produit spécifique (105), à la fois les données d'entrée et les données générées par l'enregistreur de données du dispositif de repérage (106) ou par des dispositifs supplémentaires dans le système, peuvent être traitées et transférées entre lesdites unités de système, et
selon lequel le système comprend au moins un moyen pour la lecture de ladite identité de dispositif unique du dispositif de repérage (106),
**caractérisé en ce que** le dispositif de repérage (106) comprend un boîtier étanche aux fluides et que les composants du dispositif de repérage sont conçus et fabriqués en matériaux qui permettent le nettoyage dans une machine à laver conventionnelle avec un agent de lavage à base d'eau à une température supérieure à 70 °C.

2. Système selon la revendication 1,
**caractérisé en ce que** le dispositif de repérage (106) est conçu pour accompagner ledit conditionnement (101) avec le contenu de produit spécifique pendant la même période de temps, **en ce que** : le dispositif de repérage comprend un premier dispositif de fixation (108) adapté pour être fixé à un deuxième dispositif de fixation (109) du conditionnement et/ou fixé au conditionnement à l'aide d'une sangle, d'une bande, d'une corde ou d'une chaîne et/ou fixé au moyen d'un adhésif à l'extérieur du conditionnement et/ou conditionné dans un conditionnement extérieur commun.

3. Système selon l'une quelconque des revendications précédentes,
**caractérisé en ce que** le dispositif de repérage (106) comprend en outre un ou plusieurs parmi :
• au moins un dispositif de capteur conçu pour la détection de la température ambiante, la teneur en humidité et/ou les conditions d'éclairage et/ou de rayonnement, de sorte qu'un ou plusieurs desdits paramètres puissent être enregistrés par ledit enregistreur de données ;
• une alimentation électrique intégrée avec une capacité choisie pour alimenter l'enregistreur de données tout au long de ladite période de temps ou une source d'alimentation intégrée sous la forme d'une batterie, d'une pile à combustible ou un condensateur;
• une ou plusieurs des diodes électroluminescentes (107) et/ou un générateur de sons et/ou un affichage (110), principalement pour la reproduction de la durée de vie restante estimée du contenu de produit spécifique (105) ;
• une partie de communication sous la forme d'un ou plusieurs commutateurs mécaniques capacitifs (111) ou d'un cristal piézoélectrique, qui réagit au mouvement; ou
• une marque visible disposée pour être lue visuellement.

4. Système selon l'une quelconque des revendications précédentes,
**caractérisé en ce que** le dispositif de repérage (106) est conçu pour accompagner un conditionnement (101) contenant un produit sanguin (105), et **en ce que** le dispositif indicateur intégré (110) est conçu pour montrer une indication de cassation si le produit a été exposé à une température ambiante entre une valeur maximale et une valeur minimale.

5. Système selon l'une quelconque des revendications précédentes,
**caractérisé en ce que** l'identité de dispositif unique du dispositif de repérage (106) est conçue pour être lue par un dispositif optique, tel qu'un code-barres, à l'aide d'un lecteur optique et/ou sans fil par l'intermédiaire de signaux radio, infrarouges ou capacitifs.

6. Système selon l'une quelconque des revendications 1 à 5,
**caractérisé en ce que** lesdits autres dispositifs de système dans le système incluent au moins un point d'accès (212) qui, pour l'entrée d'un nouveau conditionnement médical dans le système, est équipé de moyens pour la lecture dudit code d'identité unique (202) et d'autres informations pertinentes (203, 204) sur un conditionnement spécifique (201) et des moyens pour la transmission dudit identifiant et les informations à au moins un enregistrement de données dans une base de données incluse dans le système, et des moyens pour la lecture de ladite identité de dispositif unique d'un dispositif de repérage (206) qui accompagnera le conditionnement spécifique (201) pour la manipulation continue du système et des moyens pour la transmission de ladite identité de dispositif unique à ladite base de données pour l'association audit ou auxdits enregistrements de données, et/ou
au moins un point d'accès (212) qui, pour la manipulation de conditionnement médical déjà entré dans le système et pour mettre à jour les données sur ledit conditionnement et la position dans le système, est équipé de moyens pour la lecture de ladite identité de dispositif unique de chaque dispositif de repérage (206) accompagnant un conditionnement spécifique dont les données précédentes existent déjà en tant qu'au moins un enregistrement de données dans ladite base de données avant que ledit conditionnement (201) ne soit transmis dans le système au-delà dudit point d'accès (212), et/ou
au moins un point d'accès (212) qui, pour la récupération et la mise à jour de données concernant au moins un paramètre environnemental qui affecte la durée de vie restante d'un contenu de produit de conditionnement spécifique, est pourvu de moyens pour la lecture de toutes nouvelles données sur ledit au moins un paramètre environnemental enregistré par l'enregistreur de données sur chaque dispositif de repérage (206) accompagnant un conditionnement spécifique, dont les données précédentes existent déjà en tant qu'au moins un enregistrement de données dans ladite base de données lorsque ledit conditionnement spécifique (201) passe ledit point d'accès (212).

7. Système selon la revendication 6,
**caractérisé en ce que** le point d'accès (212) comprend un ou plusieurs parmi :
• au moins un lecteur optique (213), de préférence un scanner laser pour code-barres ou code d'accès rapide ou une caméra, pour la lecture dudit code d'identité unique (202) et d'autres informations pertinentes (203, 204) sur un conditionnement spécifique (201) ;
• au moins un lecteur sans fil (214) pour des signaux radio, infrarouges ou un relevé capacitif de ladite identité de dispositif unique d'un dispositif de repérage (206), qui accompagne un conditionnement spécifique (201) ;
• au moins un lecteur sans fil (214) pour des signaux radio, infrarouges ou un relevé capacitif des données concernant au moins un paramètre environnemental enregistré par l'enregistreur avec un dispositif de repérage (206) muni d'un conditionnement spécifique (201) ;
• au moins une liaison de communication avec un serveur, de préférence LAN (Ethernet), LAN sans fil, GSM ou GPRS, pour la transmission de données entre ledit point d'accès (212) et le serveur et/ou d'autres dispositifs de système au système ;
• au moins un dispositif informatique (215) conçu pour gérer la lecture, le traitement et la communication d'informations dans le système, et la communication avec un opérateur ;
• au moins un dispositif d'indication pour la communication avec un opérateur, comprenant de préférence une diode électroluminescente/ un panneau lumineux, un afficheur (216) et/ou un générateur de sons ;
• au moins un dispositif de communication, de préférence sous forme d'une clé capacitive ou mécanique ou d'un cristal piézoélectrique qui répond à un tapotement, qui peut être affecté par un opérateur.

8. Système selon l'une quelconque des revendications précédentes,
**caractérisé en ce que** lesdits autres dispositifs de système dans le système incluent au moins un portail (317) pour les utilisateurs du système qui peuvent communiquer avec au moins un serveur de données (318) qui stocke des données relatives à tout conditionnement médical qui est entré dans le système, lesdites données stockées dans ledit serveur de données comprenant au moins :
- un identifiant unique pour chaque conditionnement spécifique (301) qui est inséré dans le système,
- une identité de dispositif unique pour l'unité de détection spécifique (306) qui accompagne le conditionnement spécifique dans la manipulation du système,
- une date de lecture la plus récente des informations concernant le conditionnement spécifique (301) stockées dans ledit serveur de données (318),
- une spécification du dernier point d'accès (312), que ledit dispositif de repérage spécifique (306) et donc le conditionnement spécifique (301) a passé dans le système,
- une date d'expiration estimée pour un contenu de produit spécifique (305) du conditionnement spécifique (301), et
- d'autres informations pertinentes qui identifient et caractérisent le contenu de produit spécifique (305) de manière unique, pour l'utilisation des moyens nécessaires de ce contenu de produit spécifique, à quelles données les utilisateurs du système peuvent accéder à partir d'au moins un terminal utilisateur (319) connecté audit portail (317) pour les utilisateurs du système.

9. Système selon l'une quelconque des revendications précédentes,
**caractérisé en ce que** le système comprend un certain nombre de points d'accès qui sont situés au moins :
- à une sortie d'un dispositif qui fabrique ou remplit un conditionnement médical avec un contenu spécifique au produit,
- à une entrée d'un dépôt ou d'un stockage intermédiaire où sont stockés lesdits conditionnements,
- à une sortie dudit dépôt ou du magasin, et
- à une entrée d'un établissement de traitement ou de soins, dans lequel s'effectue l'administration du contenu de produit spécifique.

10. Système selon l'une quelconque des revendications précédentes,
**caractérisé en ce que** le système comprend au moins un point d'accès destiné à être utilisé dans un véhicule de transport conçu pour transporter des conditionnements d'une unité de fabrication ou de remplissage à un dépôt ou un stockage intermédiaire, ou dudit dépôt ou du magasin à une unité de traitement ou de soins de santé, dudit établissement de traitement ou de soins à un autre établissement de traitement ou de soins, de retour de l'établissement de traitement ou de soins au dépôt ou au magasin, ou de cet établissement de traitement ou de soins à un autre dépôt ou à un autre stockage intermédiaire.

11. Système selon l'une quelconque des revendications précédentes,
**caractérisé en ce que** le système comprend au moins un point d'accès destiné à être utilisé à bord d'une voiture, d'une ambulance, d'un camion, d'un camion-remorque, d'un avion, d'un hélicoptère, d'un bateau ou d'un navire, ou d'un point d'accès portable pouvant être transporté vers une position souhaitée par un opérateur.

12. Dispositif de repérage destiné à être utilisé dans un système de manipulation de conditionnements médicaux, système dans lequel chaque conditionnement individuel (101) est muni d'au moins un code d'identité unique (102) et d'informations (103, 104) comprenant au moins une spécification (103) concernant le contenu de produit conditionné et la date de conditionnement (104), le système comprenant en outre au moins un moyen pour la lecture dudit code d'identité unique (102), dans lequel le dispositif de repérage est conçu pour accompagner le conditionnement (101) avec ledit contenu de produit spécifique sur une période de temps qui s'étend de l'entrée dudit conditionnement (101) dans le système à une décision éventuelle concernant l'administration de ce contenu de produit spécifique (105) à un destinataire prévu, le dispositif de repérage (106) comprenant :
• un enregistreur de données intégré, qui est conçu pour enregistrer les données et l'heure enregistrées pendant ladite période de temps concernant au moins un paramètre environnemental affectant la durée de vie dudit contenu de produit spécifique (105),
• une fonction de calcul qui, sur la base des données et de l'heure enregistrées, calcule une valeur pour ladite durée de vie restante,
• un dispositif d'indication intégré (107), qui est conçu pour indiquer, au moins à propos du contenu de produit spécifique (105), la durée de vie restante,
• un dispositif de communication (111) pour la réception d'un signal pour activer l'indication de toute durée de vie restante avec ledit dispositif d'indication (107),
• une identité de dispositif unique,
dans lequel ladite identité de dispositif unique est lisible par le système et, dans le système, associée audit code d'identité unique (102) et auxdites autres informations pertinentes (103, 104) sur ledit conditionnement (101) pour garantir que les données relatives au contenu de produit spécifique (105), à la fois les données d'entrée et les données générées par l'enregistreur de données du dispositif de repérage (106) ou par des dispositifs supplémentaires dans le système, peuvent être traitées et transférées entre lesdites unités de système, et
le système comprenant au moins un moyen pour la lecture de ladite identité de dispositif unique du dispositif de repérage (106),
**caractérisé en ce que** le dispositif de repérage (106) comprend en outre un boîtier étanche aux fluides et que les composants du dispositif de repérage sont conçus et fabriqués en matériaux qui permettent le nettoyage dans une machine à laver conventionnelle avec un agent de lavage à base d'eau à une température supérieure à 70 °C.

13. Procédé de gestion de conditionnements médicaux, dans lequel chaque conditionnement individuel (101) est muni d'au moins un code d'identité unique (102) et d'autres informations pertinentes (103, 104), comprenant au moins une spécification (103) du contenu de produit des conditionnements et de la date de conditionnement (104), qui identifie et caractérise un contenu de produit spécifique (105) dudit conditionnement, le procédé consistant à fournir au conditionnement au moins un dispositif de repérage (106), qui est conçu pour accompagner ledit conditionnement (101) avec le contenu de produit spécifique sur une période de temps s'étendant sur toute la durée de l'entrée dudit conditionnement (101) à une décision éventuelle concernant l'administration du contenu de produit spécifique (105) à un destinataire prévu, dans lequel le dispositif de repérage (106) inclut un enregistreur de données intégré, qui est conçu pour enregistrer les données et l'heure enregistrées sur au moins un paramètre environnemental affectant la durée de vie de ce contenu de produit spécifique (105) pendant la même période, et le dispositif de repérage (106) comprend un boîtier étanche aux fluides, et les composants du dispositif de repérage sont conçus et fabriqués en matériaux qui permettent le nettoyage dans une machine à laver conventionnelle avec un agent de lavage à base d'eau à une température supérieure à 70 °C, le procédé comprend en outre les étapes consistant à :
- calculer une valeur pour ladite durée de vie restante par le dispositif de repérage (106) et sur la base des données et de l'heure enregistrées,
- indiquer, par le dispositif indicateur (107) du dispositif de repérage (106), au moins à propos du contenu de produit spécifique (105), la durée de vie restante,
- permettre la détection de toute durée de vie restante au moyen dudit dispositif indicateur (107),
- gérer et transmettre des données relatives au contenu de produit spécifique (105), à la fois les données d'entrée et les données générées par l'enregistreur de données de ce dispositif de repérage (106) entre l'appel de ce système, ayant une identité de dispositif unique qui est lisible et associée à cet identifiant unique (102) et d'autres informations pertinentes (103, 104) dudit conditionnement (101).
